Europäisches Patentamt

(19) European Patent Office

Office européen des brevets

(11) Publication number: **0 038 609**
**B1**

(12) **EUROPEAN PATENT SPECIFICATION**

(45) Date of publication of patent specification: 25.07.84

(21) Application number: 81200449.7

(22) Date of filing: 23.04.81

(51) Int. Cl.³: **C 07 C 45/49,**
**C 07 C 47/19,**
**C 07 D 307/20,**
**B 01 J 31/16**

(54) Preparation of 2-hydroxytetrahydrofuran.

(30) Priority: 23.04.80 NL 8002342

(43) Date of publication of application:
28.10.81 Bulletin 81/43

(45) Publication of the grant of the patent:
25.07.84 Bulletin 84/30

(84) Designated Contracting States:
BE CH DE FR GB IT LI NL

(56) References cited:
FR - A - 2 283 114
FR - A - 2 394 540
US - A - 3 733 362

JOURNAL OF ORGANIC CHEMISTRY, vol. 45
(1980), May, no. 11, pages 2132-2139, Easton,
GB C.V. PITTMAN et al. "Rhodium catalyzed
hydroformylation of allylalcohol"

(73) Proprietor: STAMICARBON B.V.
Postbus 10
NL-6160 MC Geleen (NL)

(72) Inventor: De Munck, Nicolaas Anthony
Bosboom Toussaintplein 45
NL-2624 DE Delft (NL)
Inventor: Scholten, Joseph Johannes F.
Sartonlaan 7
NL-6132 BC Sittard (NL)

(74) Representative: Leherte, Georges Maurice Lucien
Marie et al,
Octrooibureau DSM P.O.Box 9
NL-6160 MA Geleen (NL)

Courier Press, Leamington Spa, England.

**Description**

The invention relates to a process for the preparation of 4-hydroxybutyraldehyde and/or 2-hydroxytetrahydrofuran formed by cyclization of the first-mentioned compound.

It is known that these compounds can be obtained by reaction of allyl alcohol with carbon monoxide and hydrogen (hydroformylation) in the presence of a noble metal complex, in particular a rhodium complex. According to German Patent Application 2,538,364, the hydroformylation is performed batchwise as a homogeneous catalytic reaction. The rhodium complex is then present as a solution in an inert solvent. In this hydroformylation of allyl alcohol, by-products such as 2-hydroxy-methylacetaldehyde (formed by hydroformylation of the 2nd position of the allyl alcohol), propionaldehyde (formed by isomerization of allyl alcohol), propanol (hydrogenation product) and polymers may be formed. The primary hydroformylation product is probably 4-hydroxybutyraldehyde, which can be converted by cyclization to 2-hydroxytetrahydrofuran. Both products can be converted to 1.4-butanediol.

From US patent no. 3733362 it is known to perform the gas phase hydroformylation of olefins with carbon monoxide and a coreactant such as an alcohol, amine, water or hydrogen, using a heterogeneous catalyst comprising a Group VIII metal and a biphyllic liquid.

The invention aims at a method for the hydroformylation of allyl alcohol, which can be performed with a high selectivity and continuously, without catalyst recovery problems.

According to the invention, allyl alcohol is converted with hydrogen and carbon monoxide in the presence of a catalytically active metal complex, the reaction being performed continuously in the gas phase with a catalyst consisting of a solid porous inorganic carrier material, in the pores of which is a solution of a catalytically active metallo-organic complex in one or more ligand-forming compounds with a vapour pressure or less than 1.3 mbar under the reaction conditions.

The hydroformylation with the method according to the invention has a number of distinct advantages. The reaction can be performed continuously and the recovery of the reaction product is very simple. The selectivity towards 4-hydroxybutyraldehyde and/or the cyclic derivative thereof is high. The reaction can be performed such that the isomerization of the allyl alcohol to propionaldehyde is suppressed.

The temperature at which the reaction is performed must be high enough for both allyl alcohol and the hydroformylation products formed to be present in gaseous form. A temperature of between about 90°C and 150°C is in general appropriate. A temperature of between 100°C and 130°C is preferably applied. In this temperature range the catalyst activity is high, without there yet being any loss of metallo-organic complex.

The reaction can be carried out in either a fluidized bed or a fixed bed. The pressure at which the reaction is performed can in general be chosen between 1 and 35 bar. A pressure of between 2 and 5 bar appears most appropriate.

The molar ratio between hydrogen, allyl alcohol and carbon monoxide is in general 2 to 10:1:2 to 20. Hydrogen and carbon monoxide are applied in a molar ratio of between 1:1 and 1:5.

Unconverted allyl alcohol and the hydroformylation products formed can be isolated from the reaction mixture by condensation. The allyl alcohol can be separated by distillation and recycled to the reactor. In the reactor the main product formed is 4-hydroxybutyraldehyde. During the condensation this is almost completely converted to 2-hydroxytetrahydrofuran. 1.4-butanediol can be obtained from both compounds by hydrogenation.

The catalyst consists of a solid porous inorganic carrier material, in the pores of which is a solution of a catalytically active metallo-organic complex.

Examples of inorganic solid porous materials which may be used as carrier are silica, zeolites, activated carbon and macrorectiticular polymers. Preferably, a carrier material is used with a surface that is organophilic and that contains no groups that may promote the formation of by-products and/or deactivate the metallo-organic complex. Suitable examples are silica rendered hydrophobic by heating, or silica-alumina containing no alkali metal ions and inorganic materials such as silica, the acid or basic groups of which have been converted to inert hydrophobic groups by treatment with a suitable reagent, for instance by silanization.

A suitable type is silica rendered hydrophobic by heat treatment at a temperature of at least 700°C (see for this treatment S. Kondo et al, Journal of Colloid and Interface Science, Vol. 55 No. 2 (1976) p. 421). The material must be completely or substantially free of alkali metal ions, on the one hand to prevent sintering during the heat treatment and on the other hand to suppress the aldol condensation of aldehydes formed during the hydroformylation. Carriers that by nature contain acid or basic groups on their surface, such a silica, silica-alumina or alumina, which have been treated with a suitable reagent to convert the reactive surface groups into inert groups are also very suitable. As inertizing agent, a silane can be applied that has at least one substituent on the silicon atom that reacts with the reactive surface groups.

The dimensions of the carrier particles may vary between approximately 0.01 and 5.0 mm. In a fluid bed particles between 0.01 and 0.1 mm will preferably be used, and in a fixed bed preferably between 0.2 and 2.0 mm.

2

O 038 609

The pore volume of the carriers is, after optional preliminary treatment, in general between 0.01 and 5 cm³/gram carrier. The diameter of the pores may in general be between 2 and 2000 nm. Preferably, a carrier material is used in which at least some of the pores have a diameter of less than 10 nm. The catalyst loading may be between 0.05 and 0.95 cm³ solution per cm³ pore volume. Preferably, the loading applied is between 0.2 and 0.8 cm³ solution per cm³ pore volume.

As the central metal atom in the catalytically active metallo-organic complex, the transition metals of Groups V, VI, VII and VIII of Mendeleef's periodic system may be considered, e.g. Re, Fe, Ru, Os, Co, Rh, Ir, Ni, Pd, Pt, particularly rhodium, cobalt, ruthenium and iridium. These metals may also be applied as a mixture.

As ligands in the aforementioned metallo-organic complex, organic compounds may be considered that have in the molecule one atom of the group VB or VIB of Mendeleef's periodic system with a free electron pair, e.g. P, S, B, Te, Sb, As, besides ligands such as CO, H and $\sigma$ and $\pi$-bonded alkenes. Suitable also are e.g. the halogenides, such as Cl, Br, and H-, tin- and germanium II halogenides, and radicals such as acetate, propionate and readily replaceable ligands such as acetylacetonate, hydrogen, carbon monoxide, tetrahydrofuran and diolefine. Complexes that may be mentioned are rhodiumhydridocarbonyltris(triphenylphosphine), cobalthydridotetracarbonyl, rhodiumbis(trisphenylphosphine)carbonylchloride, rhodiumhydridobiscarbonylbis(triphenylphosphine) and rhodiumcarbonylchloride-bis(triphenylarsine).

According to the invention, compounds with a vapour pressure of less than 1.3 mbar under reaction conditions that are able to function as ligand in a transition metal complex may be used as solvent for the metal complex. These ligand-forming compounds, here also termed 'free ligand', need not be the same as the ligands present in the original transition metal complex. They may optionally be substituted for one or more ligands of the metal complex. It is in fact probable that the catalytically active metal complex will differ under operational conditions the catalytically active metal complex will differ under operational conditions from the metal compound originally dissolved. Particularly suitable as ligand-forming compound used as solvent is an organic compounds phosphorus, antimony or arsenic and particularly phosphorus processing a free electron pair, such as compounds with the formula $PR^1R^2R^3$ or $P(OR^1)(OR^2)(OR^3)$ where $R^1$, $R^2$, and $R^3$ represent aliphatic, aromatic or alkyl aromatic hydrocarbon groups with 1—20 carbon atoms. Examples are triethylphosphine, tributylphosphine, tricyclohexylphosphine, methyldiphenylphosphine, diethylphenylphosphine, triphenylphosphine, tri-p-tolylphosphine, trinaphthylphosphine, ethylenedi(dimethylphosphine), trimethylphosphite, trimethylolpropanephosphite, triphenylphosphite, triphenylarsine, phenyldimethylarsine and triphenylstibine. The high-boiling triarylphosphines are preferably used.

The concentration of the metallo-organic complex in the free ligand can vary within wide limits; the upper limit is determined by the solubility of the metallo-organic complex in the free ligand under reaction conditions, while the lower limit is determined mainly by economic and commercial considerations. The range within which the concentration can thus vary is e.g. $10^{-1}$—$10^{-5}$ mole/litre, more particularly $10^{-2}$—$10^{-4}$ mole/l. In the preparation of the catalyst, the carrier may be impregnated with a solution of the catalytically active metal complex or a precursor thereof in free ligand without other solvents. Just enough solvent is used to achieve the desired degree of loading immediately. It is however easier to use an auxiliary solvent in catalyst preparation, i.e. to impregnate the carrier with a solution of the catalytically active metal complex or a precursor thereof in a mixture of one or more free ligands with a volatile solvent, subsequently removing the volatile solvent again. By an inert volatile solvent is meant a component that does not strongly coordinate with the metallo-organic complex, that has a vapour pressure that is at least ten times higher than the vapour pressure of the ligand and that forms a homogeneous solution with the free ligand and metallo-organic complex, for example methanol, ethanol, benzene, toluene, xylene. The proportion of free ligand to inert volatile solvent is governed by the catalyst loading desired. For a loading of 0.5, for instance, just 50% of the catalyst solution obtained will consist of volatile solvent. Just enough catalyst solution is impregnated to fill the complete pore volume of the carrier material in the first instance. If the ligand is present in solid form at room temperature, the mixture consisting of metallo-organic complex, free ligand and inert volatile solvent, is heated to the temperature at which a homogeneous solution is obtained. The hot, homogeneous catalyst solution is slowly added to the carrier material, which is also hot, atmospheric oxygen being excluded and the mixture being stirred throughout. The carrier material is previously heated to a temperature at least equal to the temperature of the catalyst solution employed. The impregnation may also be performed in a vacuum. The free-flowing catalyst obtained is now freed of the volatile solvent in vacuo, by the passage of inert gas or in situ in the reactor, at a temperature at which the volatile solvent evaporates and which is preferably above the melting point of the free ligand. In this way, redistribution of the free ligand in the carrier material is already possible during the drying of the catalyst.

The invention will now be elucidated with reference to the following examples, without being restricted to the embodiments described therein.

Example I

A catalyst was prepared by impregnating 9.23 g silica (commercial product OOO-3E, Akzo Chemie

3

Nederland) with a pore volume of 0.85 cm³/g and a particle size between 0.42 and 0.50 mm with a solution of 0.148 g rhodiumhydridocarbonyltris(triphenylphosphine) in 4.04 g triphenylphosphine diluted with an equal quantity of benzene. Subsequently, the benzene was evaporated off. The degree of filling of the pores with the solution of the rhodium complex is then 50% of the catalyst thus obtained, 4.68 g was transferred to a reactor. Allyl alcohol was hydroformylated by passing a mixture of allyl alcohol, hydrogen and carbon monoxide through the reactor in a volume ratio of 1:4.76:4.76, at a pressure of 3 bar and a temperature of 90°C. The total quantity of gas was 66.3 n.ml/minute. The reaction was carried out continously for 80 hours. In total, 19.8% of the allyl alcohol applied was converted, 18.7% into 2-hydroxytetrahydrofuran and 1.1% into propionaldehyde. The selectivity of the hydroformylation was therefore 94.4%. Other products were not found. The activity of the catalyst is 3.4 n.ml allyl alcohol converted per g rhodium (as metal) per second.

Example II

A catalyst was prepared in the manner described in Example I by impregnating 8.9 g silica S with a solution of 0.133 g rhodiumhydridocarbonyltris(triphenylphosphine) in 3.22 g triphenylphosphine and benzene followed by the evaporation of the benzene. The degree of filling of the pores was 50%. Silica S is an Na-deficient silica (96 ppm Na) that is heated for 5 hours at 850°C. The surface area is 100 m²/g, the pore volume 0.99 cm³/g and the mean particle diameter $(dV/dR)_{max}$ is 17 nm. The fraction with a particle size of 1.2 to 1.7 nm was used. Of the catalyst thus obtained, 5.86 g was transferred to a reactor. A mixture of allyl alcohol hydrogen and carbon monoxide in a volume ratio of 1:7.69:7.69 was passed for 62 hours through the reactor at 90°C and a pressure of 3 bar. The total quantity of gas was 85.2 nml/minute. The conversion of allyl alcohol was 30.6%, with 95% selectivity towards 2-hydroxytetrahydrofuran and 15% towards propionaldehyde. The activity was 3.72 ml allyl alcohol converted per g Rh per second.

Subsequently, a mixture of allyl alcohol, hydrogen and carbon monoxide in a volume ratio of 1:3.81:3.81 was passed at a quantity of 90.5 nml/minute through the reactor for 62 hours at 110°C and 3 bar. The conversion was 17.9%, the selectivity towards 2-hydroxytetrahydrofuran 97.8% and the activity was 5.06 nml allyl alcohol/g Rh.s.

Example III

A catalyst was prepared in the manner described in Example I by impregnating 8.84 g silica S with a solution of 0.986 g rhodiumhydridocarbonyltris(triphenylphosphine) in 3.31 g tri-p-tolylphosphine. The degree of filling was 50%. A mixture of allyl alcohol, hydrogen and carbon monoxide in a volume ratio of 1:7.73:7.73 was passed through the reactor in which 3.1 g catalyst had been introduced at 88°C and 4 bar at a quantity of 90.5 nml/minute. The conversion was 6.3%, the selectivity towards 2-hydroxytetrahydrofuran 81% and the activity 1.91 ml allyl alcohol/g Rh.s. The duration of the test was 95 hours. With increase of the temperature to 108°C, other reaction conditions remaining the same, the conversion became 13.2%, the selectivity 79.5% and the activity 3.93 nml allyl alcohol/g Rh.s. The duration of the test was 65 hours.

Subsequently, 172.8 nml/minute of a gaseous mixture of allyl alcohol, hydrogen and carbon monoxide in a volume ratio of 1:7.50:7.50 was passed through the reactor at 108°C and 4 bar. The conversion was 26.7%, the selectivity 89.8% and the activity 11.03 nml allyl alcohol/g Rh.s. The duration of the test was 80 hours.

In all cases, propionaldehyde was the only by-product that could be demonstrated. Formation of this by-product is countered by using a sodium-deficient carrier material. In the 60 to 95 hour tests, no loss of phosphine or metallo-organic complex was observed, and the activity and selectivity remained constant.

**Claims**

1. Process for the preparation of 2-hydroxytetrahydrofuran and/or 4-hydroxybutyraldehyde by hydroformylation of allyl alcohol with hydrogen and carbon monoxide in the presence of a catalytically active metallo-organic complex, characterized in that the reaction is performed continuously in the gas phase at a temperature of between about 90°C and 150°C with a catalyst consisting of a solid porous inorganic carrier material, in the pores of which is a solution of a catalytically active metallo-organic complex in one or more ligandforming compounds with a vapour pressure of less than 1.3 mbar under the reaction conditions.

2. Process according to Claim 1, characterized in that the reaction is performed at a temperature of between 100°C and 130°C.

3. Process according to Claims 1—2, characterized in that the reaction is performed at a pressure of between 2 and 5 bar.

4. Process according to Claims 1—3, characterized in that the molar ratio between hydrogen, allyl alcohol and carbon monoxide is 2 to 10:1:2 to 10.

5. Process according to Claims 1—4, characterized in that the molar ratio between hydrogen and carbon monoxide is between 1:1 and 1:5.

# 0 038 609

6. Process according to Claims 1—5, characterized in that the carrier material has an organophilic surface that is completely free of acid or basic groups and from alkali metal ions, or substantially so.

7. Process according to Claims 1—6, characterized in that the metallo-organic complex is dissolved in a triarylphosphine.

## Revendications

1. Procédé de préparation de 2-hydroxytétrahydrofurane et/ou de 4-hydroxybutyraldéhyde par hydroformylation d'alcool allylique à l'aide d'hydrogène et d'oxyde de carbone en présence d'un complexe métallo-organique catalytiquement actif, caractérisé en ce que la réaction est exécutée en continu en phase gazeu se à une température située environ entre 90°C et 150°C, à l'aide d'un catalyseur constitué d'un matériau de support inorganique solide et poreux, dans les pores duquel se trouve une solution d'un complexe métallo-organique catalytiquement actif dans un ou plusieurs composés générateurs de ligands avec une pression de vapeur de moins de 1,3 mbar dans les conditions réactionnelles.

2. Procédé selon la revendication 1, caractérisé en ce que la réaction est exécutée à une température située entre 100°C et 130°C.

3. Procédé selon l'une des revendications 1 et 2, caractérisé en ce que la réaction est exécutée à une pression située entre 2 et 5 bars.

4. Procédé selon l'une des revendications 1 à 3, caractérisé en ce que le rapport molaire entre l'hydrogène, l'alcool allylique et l'oxyde de carbone est de 2 à 10:1:2 à 10.

5. Procédé selon l'une des revendications 1 à 4, caractérisé en ce que le rapport molaire entre l'hydrogène et l'oxyde de carbone se situe entre 1:1 et 1:5.

6. Procédé selon l'une des revendications 1 à 5, caractérisé en ce que le matériau de support a une surface organophilique entièrement ou pratiquement exempte d'acide ou de groupes basiques et d'ions de métal alcalin.

7. Procédé selon l'une des revendications 1 à 6, caractérisé en ce que le complexe métallo-organique est dissous dans une triarylphosphine.

## Patentansprüche

1. Verfahren zur Herstellung von 2-Hydroxytetrahydrofuran und/oder 4-Oxybutyraldehyd durch Hydroformylierung von Allylalkohol mit Wasserstoff und Kohlenmonoxid in Anwesenheit eines katalytisch aktiven metallorganischen Komplexes, dadurch gekennzeichnet, daß die Reaktion kontinuierlich in der Gasphase durchgeführt wird, und zwar bei einer Temperatur zwischen etwa 90°C und 150°C mit einem Katalysator, bestehend aus einem festen, porösen, anorganischen Träger, in dessen Poren sich eine Lösung eines katalytisch aktiven metallorganischen Komplexes in einer oder mehreren ligandenbildenden Verbindungen befindet, und zwar bei einem Dampfdruck von weniger als 1,3 mbar unter den Reaktionsbedingungen.

2. Verfahren nach Anspruch 1, dadurch gekennzeichnet, daß die Reaktion bei einer Temperatur zwischen 100°C und 130°C durchgeführt wird.

3. Verfahren nach den Ansprüchen 1—2, dadurch gekennzeichnet, daß die Reaktion bei einem Druck zwischen 2 und 5 bar durchgeführt wird.

4. Verfahren nach den Ansprüchen 1—3, dadurch gekennzeichnet, daß das Molverhältnis zwischen Wasserstoff, Allylalkohol und Kohlenmonoxid 2 bis 10:1:2 bis 10 beträgt.

5. Verfahren nach den Ansprüchen 1—4, dadurch gekennzeichnet, daß das Molverhältnis zwischen Wasserstoff und Kohlenmonoxid zwischen 1:1 und 1:5 beträgt.

6. Verfahren nach den Ansprüchen 1—5, dadurch gekennzeichnet, daß der Träger eine organophile Oberfläche besitzt, welche vollkommen oder im wesentlichen frei von sauren oder basischen Gruppen sowie Alkalimetallionen ist.

7. Verfahren nach den Ansprüchen 1—6, dadurch gekennzeichnet, daß der metallorganische Komplex in einem Triarylphosphin gelöst ist.